# EUROPEAN PATENT APPLICATION

(11) **EP 0 903 571 A2**
(43) Date of publication of application: **24.03.1999**
(21) Application number: 98307584.7
(22) Date of filing: 17.09.1998
(51) Int. Cl.: G01N 21/31

(54) **Apparatus and method for determining the concentration of specific substances**

(30) Priority: 19.09.1997 JP 254837/97
(71) Applicant: MATSUSHITA ELECTRIC INDUSTRIAL CO., LTD., Kadoma-shi, Osaka-fu 571 (JP)
(72) Inventor: Uchida, Shinji, Neyagawa City, 572-0807 (JP); Atsuta, Hiroshi, Katano City, 576-0054 (JP)
(74) Representative: Price, Paul Anthony King

(57) **Abstract**

An improved apparatus and method for spectroscopic determination of a concentration of specific substance, especially body fluid substances in a living body is disclosed. The apparatus for determination of the concentration of specific substance comprises a light source, a light projector for projecting a light emitted from the light source to a specimen, a plurality of light receivers for receiving the light projected by the light projector and passed through the specimen, a beam splitter for splitting the light received by the light receiver, and light detector for detection of the light with specific wavelength split by the beam splitter.

## Description

The present invention relates to an apparatus and a method for spectroscopically analyzing and determining concentrations of light-absorbing substances in specimens, such as glucose in body fluids, including blood, of human or other living bodies.

Some apparatuses for spectroscopic determination of such concentrations of specific substances are disclosed in, for example, the Japanese Laid-Open Patent Publication No. Hei 9-182739.

This prior art spectroscopic determination apparatus is so constituted that light is projected from a light source to a specimen and the light propagated through the specimen is spectroscopically analyzed to determine a concentration of a light-absorbing substance in the specimen.

The outline of this prior art apparatus is shown in FIG. Light projected by a light source 31 is converged by a collective lens 32 and emitted to a specimen 33 as, for example, the human finger. The emitted light is propagated through the specimen while scattering, then outputted therefrom. Specific wavelength components of the light passing through the specimen 33 are absorbed by the light-absorbing substance in the specimen. The light outputted from the specimen 33 is converged by a collective lens 34. A beam splitter 35 divides the light converged by the collective lens 34. Interference filters 36 and 37 permit only specific wavelength components for which the light-absorbing substances exhibit high absorbances to transmit therethrough. Photo detectors 38 and 39 output to an arithmetic unit 40 signals according to the intensity of the reached light. The arithmetic unit 40 calculates the concentrations of the light-absorbing substances on the basis of the output signals from the photo detectors 38 and 39.

Such prior art apparatus, however, provides a large unevenness in measurements of the concentration of substances in the human body fluid. This is because the fluid in the living body is not distributed uniformly. And the slightest dislocation of a spot for projecting the light on the specimen would change a path on which the light is propagated in the living body, and hence information contained in the light going out of the living body would change. In testing with the light projection spot of the living body placed on a holder, the slightest change in pressure applied on the holder could alter the flow of blood, resulting in a significant change in measurements.

### BRIEF SUMMARY OF THE INVENTION

An object of the present invention is to provide an apparatus and method for spectroscopic determination of the concentration of specific substances which is free from the aforementioned problems and allows accurate determination of concentration of specific substance, especially body fluid components.

An apparatus for determination of a concentration of specific substance according to the present invention comprises: a light source; a light projector for projecting a light emitted from the light source to a specimen; a plurality of light receivers for receiving the light projected by the light projector and passed through the specimen; a beam splitter for splitting the light received by the light receivers; and a light detector for detecting the light split by the beam splitter.

According to the present invention, a light is projected to a specimen such as living body, and a component which passed inside the specimen and was allowed to take exit from the specimen, out of the projected light, is received by a plurality of light receivers. The received light is spectroscopically analyzed to find a concentration of a light-absorbing substance contained in the specimen. Receiving the light from the specimen by a plurality of light receivers can minimize an influence on the measurement due to a dislocation of a spot on which the light is projected, a change in contact between the specimen and the light projector or light receivers, or a change in pressure applied on the specimen. Therefore, the present invention is especially useful for analysis of the body fluid substances in the living body. The provision of a plurality of light receivers gives information on a plurality of light components propagated through different paths in the specimen and so permits more accurate determination of the concentration of an unevenly distributed body fluid substance in the living body.

In a preferred mode of the present invention, the light projector or light receivers are provided with optical fibers.

In another preferred mode of the present invention, a light-outgoing terminal of the light projector and light-incoming terminals of the light receivers are arranged on the same plane.

It is preferable that a plurality of the light-incoming terminals are arranged on a plurality of concentric circles, respectively, with the light-outgoing terminal on the center of the circles.

It is further preferable that the light-incoming terminals arranged on the larger circle are larger in area than the light-incoming terminals arranged on the smaller circle.

It is also preferable that the apparatus further comprises a calculation means for calculating the concentration of the specific component contained in the specimen on the basis of the difference or ratio in intensity of the received light between the sets of light-incoming terminals arranged on the different circles.

A method for measurement of a concentration of specific substances includes the steps of:
projecting a light to a specimen;
receiving the light projected and passed through the specimen by a plurality of light receivers;
detecting the intensity of a light component with specific wavelength range, out of the light received by the respective light receivers; and
calculating a concentration of specific substance contained in the specimen on the basis of the difference or ratio in light intensity between the light components obtained by the light receivers.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEWS OF THE DRAWINGS

FIG. 1 is a schematic diagram showing the configuration of an apparatus for determination of a concentration of specific substance in an embodiment of the present invention.

FIG. 2 is a schematic diagram showing a configuration of a principal portion of the same apparatus.

FIG. 3 is a schematic diagram showing a configuration of an apparatus for determination of a concentration of specific substance in another embodiment of the present invention.

FIG. 4 is a top view of a measuring plane of the same apparatus.

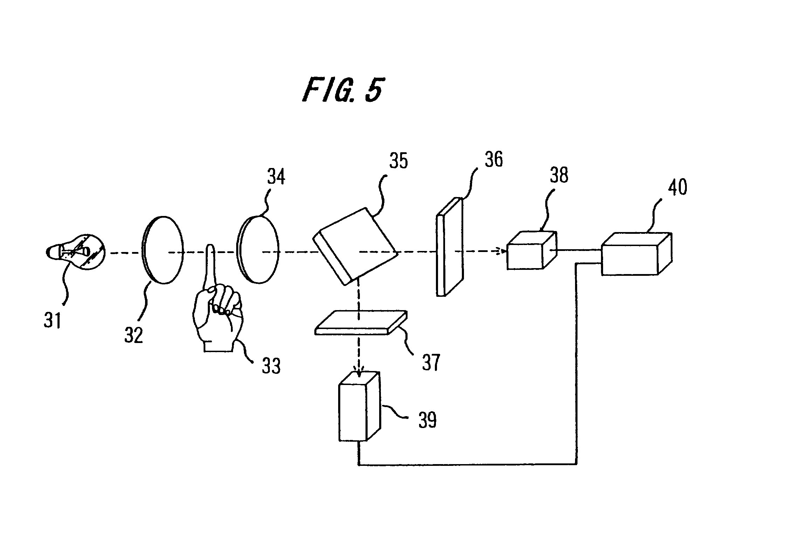

FIG. 5 is a schematic diagram of a prior art apparatus for spectroscopic determination of body fluid components.

### DETAILED DESCRIPTION OF THE PREFERRED EMBODIMENTS

Hereafter, preferred embodiments of the present invention will be described in detail with reference to the attached drawings.

### EXAMPLE 1

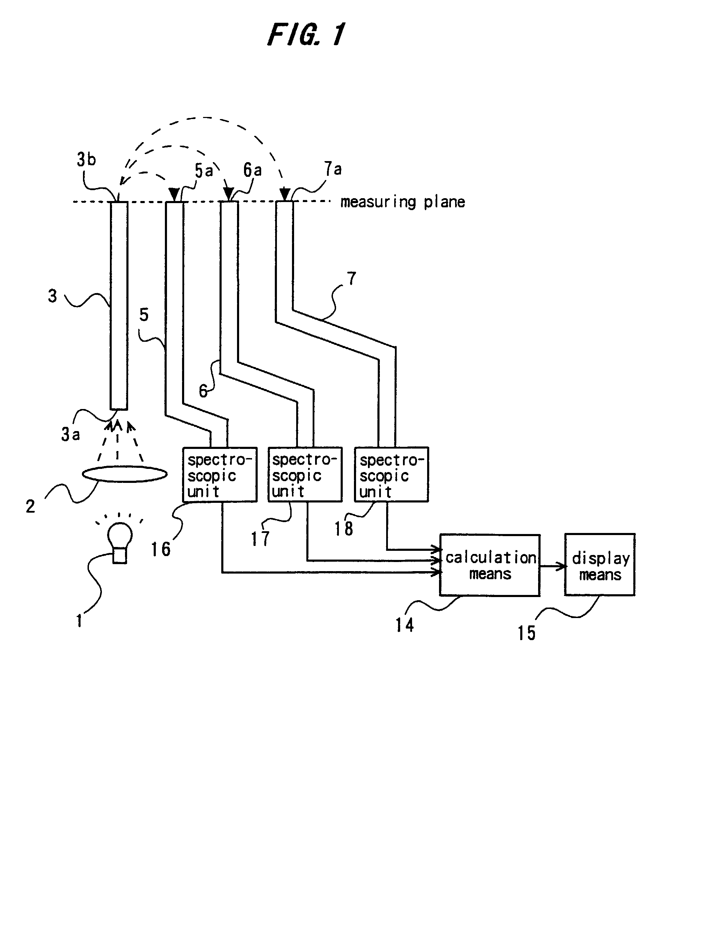

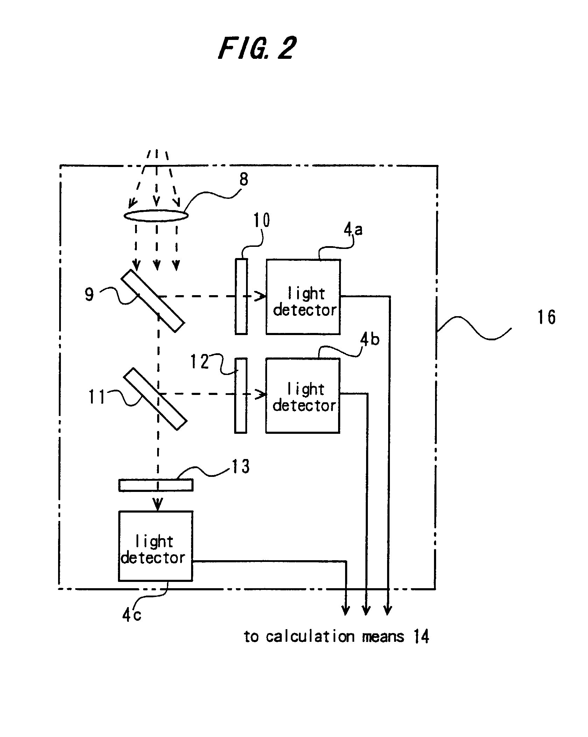

FIG. 1 and FIG. 2 show the outline of the apparatus for determination of a concentration of body fluid substance of the present embodiment.

A light source 1 is provided with a halogen lamp capable of emitting light rays with wavelength range of 400 to 2,200 nm, for example. The light emitted from the light source 1 is converged by a collective lens 2 and sent into an optical fiber 3 from an inlet terminal 3a thereof. This optical fiber 3 is made of such materials as quartz. Then, the light is propagated through the optical fiber and sent out from a light-outgoing terminal 3b.

Optical fibers 5, 6 and 7 are so arranged that light-incoming terminals 5a, 6a and 7a are placed on the same plane provided with the light-outgoing terminal 3b of the optical fiber 3. This plane is hereinafter referred to as "a measuring plane". Here, the light-incoming terminals 5a, 6a and 7a are placed each at different distances from the light-outgoing terminal 3b. The measuring plane is applied closely on a specimen such as a living body.

The light-incoming terminals 5a, 6a and 7a receive the light rays which have been propagated through and returned from the specimen. Entering the light-incoming terminal 5a, the light is passed through the optical fiber 5 and sent into a spectroscopic unit 16. The light rays entered the light-incoming terminals 6a and 7a are sent into spectroscopic units 17 and 18, respectively. The spectroscopic units 16, 17 and 18 put the light rays from the optical fibers 5, 6 and 7 to a spectroscopic analysis.

The spectroscopic unit 16 is outlined in FIG. 2. A lens 8 converts the light emitted from the optical fiber 5 into parallel ray. Beam splitters 9 and 11 split the light ray transmitted through the lens 8 in specific wavelength ranges components. For example, the beam splitter 9 reflects components with a wavelength range from 600 to 900 nm and transmits others. The beam splitter 11 reflects components with a wavelength range from 900 to 1,200 nm and transmits others. Interference filers 10, 12 and 13 selectively transmit specific wavelength components out of the split light rays. The interference filters 10, 12 and 13 are so set as to transmit wavelength components which are absorbed especially by the light-absorbing substances to be determined. Photo detectors 4a, 4b and 4c detect the components transmitted through the interference filters 10, 12 and 13, respectively, and output signals according to the detected light intensity. In a case the wavelength ranges which are reflected by the beam splitters 9 and 11 are those indicated above, the photo detectors 4a, 4b and 4c are provided with Si, InGaAs and Ge, respectively.

The spectroscopic units 17 and 18, configured the same way as the spectroscopic unit 16, analyze the light rays emitted from the optical fibers 6 and 7, respectively, and output signals to a calculation means 14.

The calculation means 14 calculates the concentrations of the substances to be determined on the basis of the input signals from the spectroscopic units 16, 17 and 18. A display means 15 displays the obtained results. Here, the calculation means 14 works out the concentrations of the specific substances on the basis of the intensities of the lights received from the light-incoming terminals 6a and 7a with reference to the signals of the light received from the light-incoming terminal 5a which is the nearest to the light-outgoing terminal 3b. In this way, interfering factors on the measurement such as a change in amount of the light projected and a change in position of projection spot can be eliminated.

Furthermore, provision of a plurality of light receivers provides information from different light rays which have returned after propagating through a specimen in different paths and thus permits determination with still higher accuracy of the concentration of body fluid substances of the living body, in particular. Provision of three or more light receivers, for example, can pick out and eliminate abnormal values due to a faulty measuring practice.

Of a plurality of light-incoming terminals on the measuring plane, one nearer the light-outgoing terminal receives more light that has been propagated near the surface of the specimen, while the more distant the light-incoming terminal is from the light-outgoing terminal, the larger the component of the light propagated deeper in the specimen. With specimens such as the living body in which the distribution of a substance to be determined is different in the direction of depth from the measuring plane, therefore, it is possible to obtain information in the direction of depth.

In the present example, three light receivers are provided. The number of light receiver can be changed as necessary. The respective returned light is separated into three wavelength ranges. This number also may be set as necessary. The means for splitting returned rays also may be a diffraction grating as well as the above-mentioned beam splitters, interference filters and photo detectors. Needless to say, the number of optical fibers is not limited, either.

The light source may be a light-emitting device, for example. The light detection means may be a photo diode array.

In the present example, the apparatus is of the reflection type with the light projector and light receivers arranged to face the specimen from the same direction. The apparatus may be of the transmission type in which the light passed through the specimen is received.

### EXAMPLE 2

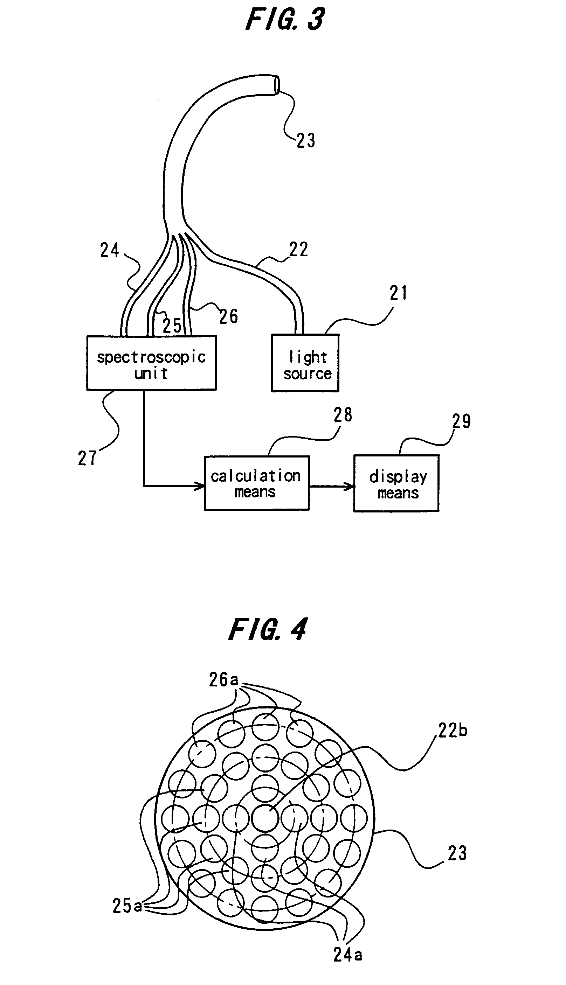

FIG. 3 illustrates the outline of an apparatus for determination of the concentrations of body fluid substances of the present embodiment.

A light source 21 is the same halogen lamp as in Example 1 which is capable of emitting light with a wavelength range from of 400 to 2,200 nm. The light emitted from the light source 21 is propagated through an optical fiber 22, then sent out from a terminal (hereinafter, referred to as "an light-outgoing terminal") located on a measuring plane 23. Optical fibers 24, 25 and 26 are connected to a spectroscopic unit 27, each by one end. They are each branched off in a plurality of lines, and the other ends, that is, the light-incoming terminals, of the plurality of branched lines are arranged on the measuring plane 23.

On the measuring plane 23, a plurality of light-incoming terminals 24a, 25a and 26a of the optical fibers 24, 25 and 26 are arranged on concentric circles with a light-outgoing terminal 22b of the optical fiber 22 on the center as shown in FIG. 4. In measurement, this measuring plane 23 is applied on a specimen such as a living body.

The light-incoming terminals 24a, 25a and 26a receive the light projected from the light-outgoing terminal 22b and propagated through the specimen. The light is received at the light-incoming terminals 24a, 25a and 26a, and converged. The optical fibers 24, 25 and 26 then transmit the converged light rays to a spectroscopic unit 27.

The spectroscopic unit 27 is the same as the one used in the analysis apparatus in Example 1 and is provided with beam splitters, interference filters and photo detectors. The spectroscopic unit 27 separates in specific wavelength ranges the light rays received on the measuring plane 23 by the optical fibers 24, 25 and 26, respectively, and outputs to a calculation means 28 signals according to the intensities of light rays in respective wavelength ranges.

The calculation means 28 calculates the concentrations of specific substances to be determined on the basis of output signals from the spectroscopic unit 27. A display means 29 displays the obtained results.

The light from the light-outgoing terminal 22b of the optical fiber 22 is projected on the specimen, propagated therethrough and returned to the spectroscopic unit 27 through the optical fibers 24, 25 and 26. The light received from an light-incoming terminal more distant from the light-outgoing terminal 22b of the optical fiber 22 contains a component penetrated deeper into the specimen. In other words, the light-incoming terminal 26a of the optical fiber 26, for example, can receive the light which has penetrated deeper in the specimen than the terminal 25a of the optical fiber 25. Meanwhile, an amount of light received per one optical fiber decreases with increase in the distance between the light-incoming terminal and the light-outgoing terminal. The apparatus for determination of concentrations in the present example has on the measuring plane a plurality of light-incoming terminals arranged on concentric circles with the light-outgoing terminal of the optical fiber as center. The larger the circle, the larger the number of light-incoming terminals. Thus, the decrease in the amount of light received from the optical fibers can be compensated. That also provides information from more spots at the same depth and compensates the loss in the quantity of information due to the decrease in amount of light received.

The amount of light received can also be increased by enlarging the diameter of the light-incoming terminal on an circle around the light-outgoing terminal.

An arrangement of a plurality of light-incoming terminals on concentric circles as in the apparatus in the present example ensures that there will be no decrease in the amount of light received, because even if the measuring plane is applied lop-sidedly on the specimen with a part of the light-incoming terminals not closely put on the specimen, the light-incoming terminals on the other side of the circles come in close contact with the specimen. Therefore, measurement can be made with high accuracy even if the contact between the measuring plane and the specimen is not uniform.

An arrangement of light-incoming terminals and the light-outgoing terminal on the same plane with the light-incoming terminals on concentric circles makes measurement easy, for example, when the measuring plane is applied on the specimen. A curved surface of the measuring plane in particular can further facilitate the contact with the specimen without causing pain on the living body.

Use of a plurality of light receivers can eliminate the possibility of signals fluctuating as when the living body makes a delicate move in measurement and thus allows determination with high accuracy.

As set forth above, the present invention can provide an apparatus which permits reliable determination of the concentrations of specific substances with a high accuracy. It is possible to provide an apparatus which can determine especially the body fluid substances in the living body with ease and high accuracy.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

## Claims

1. An apparatus for measurement of a concentration of specific substance comprising:
a light source; a light projector for projecting a light emitted from said light source to a specimen;
a plurality of light receivers for receiving the light projected by said light projector and passed through said specimen;
a beam splitter for splitting the light received by said light receivers; and
a light detector for detecting the light split by said beam splitter.

2. The apparatus for measurement of a concentration of specific substance in accordance with claim 1, wherein said light projector or said light receivers are provided with optical fibers.

3. The apparatus for measurement of a concentration of specific substance in accordance with claim 1, wherein a light-outgoing terminal of said light projector and light-incoming terminals of said light receivers are arranged on the same plane.

4. The apparatus for measurement of a concentration of specific substance in accordance with claim 3, wherein a plurality of said light-incoming terminals are arranged on a plurality of concentric circles, respectively, with said light-outgoing terminal provided on the center of said circles.

5. The apparatus for measurement of a concentration of specific substance in accordance with claim 4, wherein the light-incoming terminals arranged on the larger circle are larger in area than the light-incoming terminals arranged on the smaller circle.

6. The apparatus for measurement of a concentration of specific substance in accordance with claim 3, further comprising a calculation means for calculating the concentration of the specific component contained in said specimen on the basis of the difference or ratio in intensity of the received light between the sets of light-incoming terminals arranged on the different circles.

7. A method for measurement of a concentration of specific substances includes the steps of:
projecting a light to a specimen;
receiving the light projected and passed through the specimen by a plurality of light receivers;
detecting an intensity of a light component in specific wavelength range out of the light received by the respective light receivers; and
calculating a concentration of specific substance contained in said specimen on the basis of the difference or ratio in light intensity between said light components obtained by said light receivers.
